# FASCICULE DE BREVET EUROPEEN

(11) **EP 2 822 515 B1**
(45) Date de publication et mention de la délivrance du brevet: **11.05.2016**
(21) Numéro de dépôt: 13712884.9
(22) Date de dépôt: 04.03.2013
(51) Int. Cl.: A61F 5/02, A61F 5/03

(54) **CEINTURE DE SOUTIEN LOMBAIRE**
LUMBALER STÜTZGURT
LUMBAR SUPPORT BELT

(30) Priorité: 08.03.2012 FR 1252094
(43) Date de publication de la demande: 14.01.2015
(73) Titulaire: Gibaud, 42000 Saint Etienne (FR)
(72) Inventeur: ANGLADA, Gérard, F-42000 Saint Etienne (FR); GIRARD, Frédéric, F-75009 Paris (FR)
(74) Mandataire: Delorme, Nicolas
(86) Numéro de dépôt international: PCT/FR2013/050448
(87) Numéro de publication internationale: WO 2013/132180

(56) Documents cités:
- FR-A1- 2 952 807
- GB-A- 2 482 309
- US-A- 5 241 704
- US-A- 5 499 965
- US-A- 5 560 046

## Description

La présente invention concerne une ceinture de soutien lombaire.

Une telle ceinture est destinée aux personnes souffrant de lombalgie, notamment. Lorsqu'elle est correctement positionnée, la ceinture permet d'une part de diminuer la douleur ressentie par l'utilisateur et d'autre part de maintenir l'utilisateur dans une bonne position, en particulier grâce à la contention de sa région lombaire et de sa région abdominale.

Le document US 5 241 704 décrit une telle ceinture, comportant notamment les caractéristiques énoncées dans le préambule de la revendication 1 annexé.

La plupart des ceintures existantes donnent généralement satisfaction, mais peuvent néanmoins présenter tout ou partie des inconvénients ci-après.

Certaines ceintures sont d'un positionnement malaisé. Or, un mauvais positionnement de la ceinture est préjudiciable dans la mesure où celui-ci peut conduire à une accentuation d'un défaut de posture de l'utilisateur et peut ainsi entraîner l'aggravement de la pathologie dont souffre cet utilisateur.

Par ailleurs, la mise en place de certaines ceintures peut s'avérer difficile car l'utilisateur, du fait de sa pathologie, peut éprouver de grandes difficultés à produire l'effort nécessaire à la traction de la ceinture. Il en découle que la pression exercée par la ceinture sur la zone lombaire de l'utilisateur peut ne pas être suffisamment élevée pour que la ceinture soit efficace, notamment pour produire un effet antalgique satisfaisant.

Un autre problème réside dans le fait que certaines ceintures sont peu confortables ou le deviennent car elles ne restent pas correctement positionnées. Il s'ensuit une réticence du patient à porter la ceinture qui lui a été prescrite, la pathologie du patient ne pouvant donc pas être traitée par ce moyen.

La présente invention vise à remédier aux inconvénients mentionnés ci-dessus.

A cet effet, l'invention concerne une ceinture de soutien lombaire présentant un axe de symétrie médian transversal et qui comprend :
- un dossard destiné à être placé contre le dos d'un utilisateur et comportant deux éléments dorsaux distincts qui sont agencés sensiblement selon un V dont la pointe est dirigée vers le bas, lorsque la ceinture est à plat et à l'état non contraint, et qui sont reliés par un élément de liaison comprenant au moins une première et une deuxième bandes de textile élastique formant les deux branches d'un V dont la pointe est dirigée vers le bas, chaque bande possédant un bord médial qui, sensiblement confondu avec l'axe médian transversal, est fixé au bord médial de l'autre bande, et un bord latéral fixé au voisinage du bord médial de l'élément dorsal correspondant ;
- deux branches de textile élastique destinées à être positionnées de part et d'autre de la partie inférieure du tronc de l'utilisateur, chacune desdites branches étant fixée par son bord médial à un bord latéral d'un élément dorsal et présentant une partie extrême latérale, les parties extrêmes latérales des deux branches étant pourvues de moyens d'accrochage complémentaires pour pouvoir être assemblées lorsqu'elles sont placées l'une sur l'autre en regard de la zone abdominale de l'utilisateur, les deux branches et le dossard formant un V aplati où les parties extrêmes latérales des branches sont situées au-dessus du dossard, lorsque la ceinture est à plat et à l'état non contraint ;
- deux sangles de textile élastique situées du côté extérieur de la ceinture et agencées selon un V dont la pointe est dirigée vers le bas, chaque sangle possédant d'une part un bord médial qui, sensiblement confondu avec l'axe médian transversal, est fixé au bord médial de l'autre sangle, et d'autre part un bord latéral fixé sur la branche correspondante, les deux sangles étant fixées dans leur zone médiale sur la zone médiale de l'élément de liaison, en partie inférieure de celui-ci.

Ces dispositions de la ceinture selon l'invention se traduisent par une transformation de la poussée exercée sensiblement horizontalement par l'utilisateur lors de la mise en tension en une poussée inclinée du bas vers le haut et vers l'avant.

De façon concrète, du fait notamment de l'agencement en V des différents constituants de la ceinture, des parties extrêmes latérales des branches situées au-dessus du dossard et de la fixation des sangles en partie inférieure du dossard, lorsqu'un utilisateur tire sur les parties extrêmes latérales des branches, il se produit :
- une ouverture du dossard, c'est-à-dire une augmentation de l'angle du V formé par les deux éléments dorsaux ;
- une poussée ascendante sur la ceinture, dans la zone dorsale, tendant à déplacer le dossard vers le haut ;
- un appui exercé par la ceinture au niveau de la partie inférieure du dossard, vers l'avant.

La combinaison de ces effets contribue à atténuer la charge sur les segments bas du rachis lombaire (L3/L4/L5, c'est-à-dire troisième, quatrième et cinquième vertèbres lombaires) et sur les segments intervertébraux bas (L4/L5 et L5/S1 où se situe le disque vertébral le plus atteint par les lombalgies, S1 étant la première vertèbre sacrée).

Ainsi, la structure et la géométrie de la ceinture selon l'invention permettent d'obtenir une pression importante dans une partie appropriée de la zone lombaire, donc une excellente efficacité, sans nécessiter un effort important de la part de l'utilisateur.

En plus d'être facile à mettre en place, cette ceinture est simple à positionner de façon correcte. En effet, les deux éléments dorsaux peuvent être naturellement placés de part et d'autre de la colonne vertébrale, de façon symétrique.

Par ailleurs, la structure du dossard en deux parties - les deux éléments dorsaux - reliées par un élément de liaison textile donc relativement souple présente un certain nombre d'avantages par rapport à un dossard en une seule pièce relativement rigide :
- cela permet d'assurer un maintien contre le dos du patient sur sensiblement toute la surface du dossard, même si le patient présente de petites asymétries morphologiques ;
- cela permet de conserver ce contact contre le dos du patient y compris lors de certains mouvements du patient, comme une rotation autour de l'axe de la colonne vertébrale, puisque les deux éléments dorsaux possèdent une certaine liberté de mouvement l'un par rapport à l'autre ;
- cela améliore le confort de port, en particulier pour des patients très minces ou maigres dont les apophyses épineuses dorsales et lombaires viennent en appui contre l'élément de liaison souple, ce qui occasionne moins de gêne qu'avec un dossard rigide dans cette zone médiane.

Chacun des deux éléments dorsaux est de préférence semi rigide. En d'autres termes, il possède une rigidité suffisante pour jouer son rôle de maintien et d'appui comme exposé plus haut, à l'inverse d'une sangle souple, mais peut tout de même être légèrement déformé élastiquement pour se conformer à la morphologie du porteur sous l'effet de la traction exercée sur les branches.

Selon une réalisation possible, le dossard comprend au moins un premier ensemble de deux barres de rigidification montées chacune sur un élément dorsal, et agencées selon un V dont la pointe est dirigée vers le bas.

Le dossard peut de plus comprendre un deuxième ensemble de deux barres de rigidification montées chacune sur un élément dorsal, et agencées selon un V dont la pointe est dirigée vers le bas, les barres du deuxième ensemble étant disposées latéralement par rapport aux barres du premier ensemble - c'est-à-dire plus éloignées de l'axe médian transversal de la ceinture.

Par exemple, l'angle α1 formé par le V du premier ensemble de barres est compris entre 6 et 8°, et l'angle α2 formé par le V du deuxième ensemble de barres est compris entre 8 et 10°, lorsque la ceinture est à plat et à l'état non contraint. On peut par exemple avoir α2 > α1.

On peut prévoir que les première et deuxième bandes de l'élément de liaison soient situées en portion supérieure de l'élément de liaison et que l'élément de liaison comprenne, en portion inférieure, une troisième et une quatrième bandes de textile élastique formant les deux branches d'un V dont la pointe est dirigée vers le bas, chacune des troisième et quatrième bandes possédant un bord médial qui, sensiblement confondu avec l'axe médian transversal, est fixé au bord médial de l'autre bande, et un bord latéral fixé au bord médial de l'élément dorsal correspondant.

Ceci permet d'obtenir plus facilement la nervosité requise et de simplifier le montage. Il peut également en résulter une plus grande robustesse. Typiquement, les portions inférieure et supérieure peuvent correspondre chacune sensiblement à la moitié de la hauteur de l'élément de liaison.

Avantageusement, chaque branche peut être formée par l'assemblage de façon décalée d'au moins deux bandes sensiblement rectangulaires, de sorte que la largeur de la branche diminue depuis l'élément dorsal en direction de la partie extrême latérale.

Cet agencement permet de faciliter la confection. En effet, de cette façon, il est possible de donner à la ceinture sa forme « en trapèze » adaptée à la morphologie d'une personne sans avoir recours à des opérations de découpe du contour qui nécessiteraient de réaliser ensuite une bordure. Le recours aux ultra sons n'est pas non plus nécessaire.

Les sangles peuvent de préférence être positionnées sur la ceinture de sorte à venir en appui sur les crêtes iliaques de l'utilisateur, en position d'utilisation de la ceinture. Ceci procure un effet de coincement qui contribue à provoquer le mouvement ascendant du dossard.

En outre, les parties extrêmes latérales des branches peuvent comporter des moyens de réception des doigts de l'utilisateur qui, lorsque la ceinture est à plat et à l'état non contraint, sont disposés de façon inclinée par rapport à l'axe médian transversal d'un angle β compris entre 20 et 40°, par exemple voisin de 30°, du bas vers le haut lorsque l'on s'éloigne dudit axe.

On décrit à présent, à titre d'exemple non limitatif, un mode de réalisation possible de l'invention, en référence aux figures annexées :
La figure 1 est une vue d'une ceinture de soutien lombaire selon l'invention, à plat et à l'état non contraint ;
La figure 2 représente le dossard seul de la ceinture de la figure 1 ;
La figure 3 représente uniquement le dossard et les sangles de la ceinture de la figure 1 ;
Les figures 4 et 5 montrent la ceinture de la figure 1 au début de la mise en place sur un utilisateur représenté respectivement de dos et de profil ;
Les figures 6 et 7 sont des vues analogues aux figures 4 et 5, respectivement, lorsque l'utilisateur exerce une traction sur les branches de la ceinture.
Les figures 8 et 9 sont des vues analogues aux figures 6 et 7, respectivement, et montrent schématiquement les forces exercées par la ceinture sur l'utilisateur ;
Les figures 10 et 11 représentent une ceinture à plat, respectivement à l'état non contraint et lorsqu'elle est mise en traction.

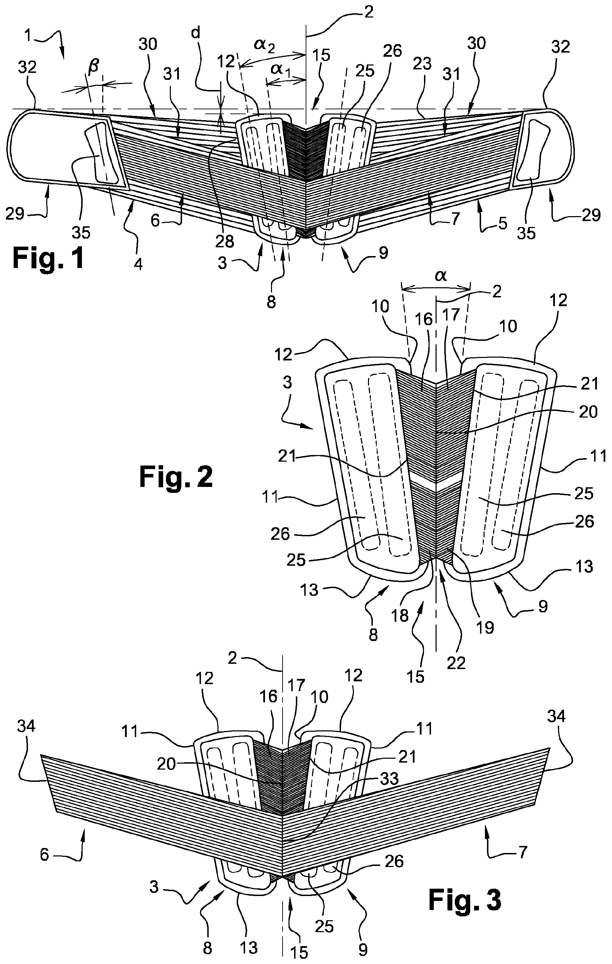

La figure 1 illustre une ceinture de soutien lombaire 1 selon l'invention, lorsqu'elle est posée à plat et est à l'état non contraint, c'est-à-dire en particulier lorsqu'aucune traction n'est exercée sur elle.

La ceinture 1 possède un axe de symétrie médian transversal 2.

L'expression « axe transversal » s'entend par rapport à la ceinture. Il s'agit, lorsque la ceinture est en position d'utilisation - c'est-à-dire portée par un utilisateur - d'un axe sensiblement orthogonal au plan transversal de la personne, soit globalement vertical. Les termes « médial » et « latéral » sont employés en référence à cet axe médian 2. Un élément sera qualifié de « médial » s'il est situé plus près de cet axe qu'un autre élément, alors qualifié de « latéral ». Les termes « haut », « bas », « supérieur » et « inférieur » sont employés par rapport à cet axe médian 2 dans la position qu'occupe la ceinture lorsqu'elle est portée.

La ceinture 1 comprend pour l'essentiel un dossard 3, représenté seul sur la figure 2, deux branches 4, 5, et deux sangles 6, 7.

Le dossard 3 est destiné à être placé contre le dos d'un utilisateur. Il comporte deux éléments dorsaux 8, 9 distincts se présentant par exemple sous la forme de coussins en mousse contrecollée.

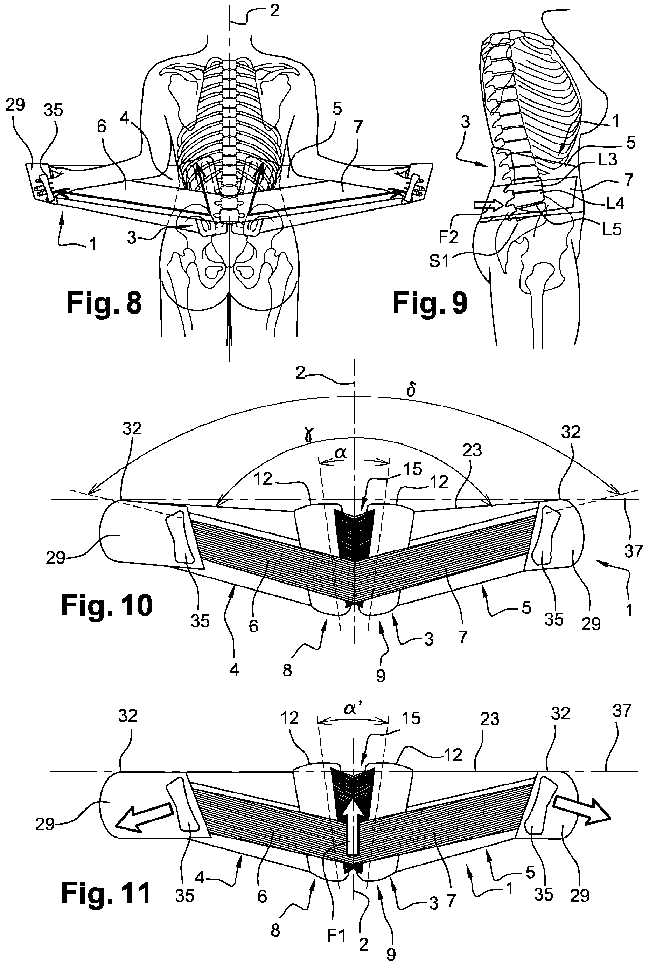

Les éléments dorsaux 8, 9 sont agencés sensiblement selon un V dont la pointe est dirigée vers le bas. L'angle α formé par le V peut être de l'ordre de 5 à 10°, et plus spécifiquement de 5 à 8°, lorsque la ceinture est à plat et à l'état non contraint (voir figure 10). En d'autres termes, l'écartement entre les deux éléments dorsaux 8, 9 est plus important en partie supérieure du dossard 3 qu'en partie inférieure. Toutefois, de préférence, les éléments dorsaux 8, 9 ne sont pas jointifs.

Chaque élément dorsal 8, 9 comporte un bord médial 10 - plus proche de l'axe 2 - et un bord latéral 11 - plus éloigné de l'axe 2, ainsi qu'un bord supérieur 12 et un bord inférieur 13. Les bords médial 10 et latéral 11 d'un élément dorsal 8, 9 sont de préférence sensiblement rectilignes. Le bord supérieur 12 d'un élément dorsal 8, 9 peut être droit ou courbe et convexe en étant légèrement incliné vers le haut en direction de l'axe 2. Le bord inférieur 13 d'un élément dorsal 8, 9 peut être droit ou courbe et convexe en étant légèrement incliné vers le bas en direction de l'axe 2.

Les éléments dorsaux 8, 9 sont reliés par un élément de liaison 15. Comme illustré plus particulièrement sur la figure 2, l'élément de liaison 15 peut comprendre :
- dans sa moitié supérieure, une première et une deuxième bandes 16, 17 de textile élastique formant les deux branches d'un V dont la pointe est dirigée vers le bas ;
- et dans sa moitié inférieure une troisième et une quatrième bandes 18, 19 de textile élastique formant les deux branches d'un V dont la pointe est dirigée vers le bas.

Chaque bande 16-19 possède un bord médial 20 qui est sensiblement confondu avec l'axe médian transversal 2 de la ceinture 1 et qui est fixé au bord médial 20 de l'autre bande située en regard - horizontalement. De plus, chaque bande possède un bord latéral 21 fixé au voisinage du bord médial 10 de l'élément dorsal 8, 9 correspondant.

L'espace entre les deux éléments dorsaux 8, 9 étant sensiblement triangulaire, la longueur des troisième et quatrième bandes 18, 19 - orthogonalement à l'axe 2 - est inférieure à la longueur des première et deuxième bandes 16, 17.

On peut prévoir de façon optionnelle une échancrure 22 en partie inférieure de l'élément de liaison 15, c'est-à-dire dans les troisième et quatrième bandes 18, 19, pour une meilleure adaptation à la morphologie de l'utilisateur et donc un confort accru.

Par ailleurs, le dossard 3 comprend, dans la réalisation représentée :
- un premier ensemble de deux barres de rigidification 25 montées chacune sur un élément dorsal 8, 9, et agencées selon un V dont la pointe est dirigée vers le bas ;
- et un deuxième ensemble de deux barres de rigidification 26 montées chacune sur un élément dorsal 8, 9, et agencées selon un V dont la pointe est dirigée vers le bas, les barres 26 du deuxième ensemble étant disposées latéralement par rapport aux barres 25 du premier ensemble.

Les barres de rigidification 25, 26 peuvent être typiquement réalisées en acier ressort. Elles présentent de préférence une légère courbure pour s'adapter à la lordose de l'utilisateur de la ceinture 1. Les barres 25, 26 peuvent être logées dans des fourreaux prévus sur les éléments dorsaux 8, 9, mais d'autres modes d'assemblage peuvent être envisagés.

L'angle α1 formé par le V du premier ensemble de barres 25 est par exemple compris entre 6 et 8°, tandis que l'angle α2 formé par le V du deuxième ensemble de barres est compris entre 8 et 10°, lorsque la ceinture 1 .est à plat et à l'état non contraint.

Les branches 4, 5 sont destinées à être positionnées de part et d'autre de la partie inférieure du tronc de l'utilisateur. Elles sont réalisées en textile élastique de façon à apporter la contention appropriée.

Chacune des branches 4, 5 est fixée par son bord médial 28 à un bord latéral 11 d'un élément dorsal 8, 9 et présente une partie extrême latérale 29. Les parties extrêmes latérales 29 des deux branches 4, 5 sont pourvues de moyens d'accrochage complémentaires (non représentés), par exemple du type velours et crochet d'un système Velcro ®. Ainsi, les deux branches 4, 5 peuvent être assemblées lorsqu'elles sont placées l'une sur l'autre en regard de la zone abdominale de l'utilisateur.

De préférence, chaque branche 4, 5 peut être formée par l'assemblage d'au moins deux bandes 30, 31 sensiblement rectangulaires. Ces bandes 30, 31 sont assemblées en étant décalées l'une par rapport à l'autre, angulairement et/ou transversalement, comme on le voit sur la figure 1. Ainsi, on peut donner aux branches 4, 5 une forme non rectangulaire, la largeur de la branche diminuant depuis l'élément dorsal 3 en direction de la partie extrême latérale 29 de la branche. Cette partie extrême latérale 29 peut être une pièce rapportée sur les bandes 30, 31.

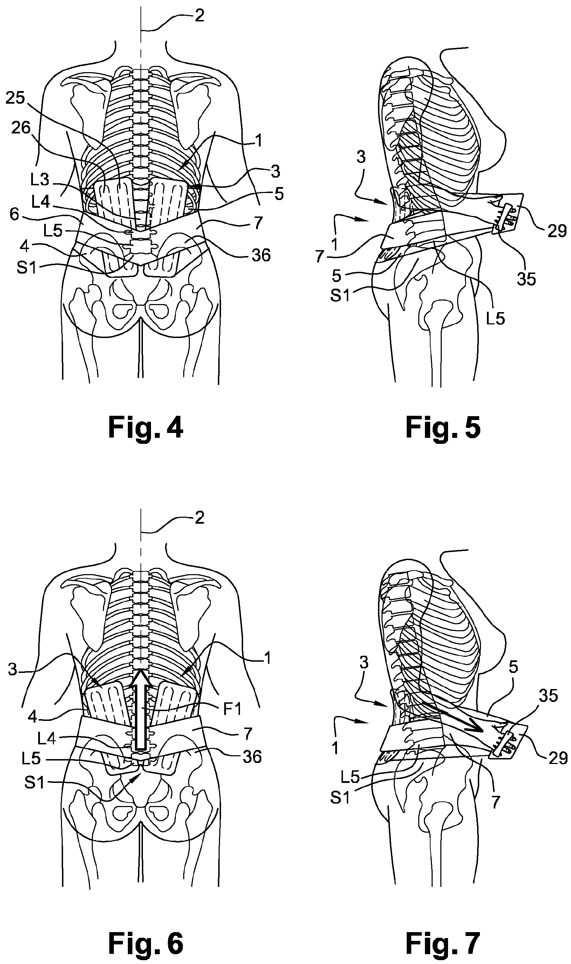

Par ailleurs, les parties extrêmes latérales 29 des branches 4, 5 comprennent des moyens de réception des doigts de l'utilisateur, se présentant ici sous la forme de lanières 35 fixées à leurs extrémités et sous lesquelles l'utilisateur peut passer ses doigts comme illustré sur la figure 5. Dans la réalisation représentée sur la figure 1, lorsque la ceinture 1 est à plat et à l'état non contraint, les lanières 35 sont disposées de façon inclinée par rapport à l'axe médian transversal 2 d'un angle β compris entre 20 et 40°, par exemple voisin de 30°, du bas vers le haut lorsque l'on s'éloigne dudit axe 2.

De plus, lorsque la ceinture 1 est à plat et à l'état non contraint, les deux branches 4, 5 et le dossard 3 forment un V aplati où les parties extrêmes latérales 29 des branches 4, 5 sont situées au-dessus du dossard 3. En d'autres termes, comme illustré sur la figure 1, il existe un décalage d selon l'axe 2 entre le bord supérieur 32 des parties extrêmes latérales 29 des branches 4, 5 et le bord supérieur 12 des éléments dorsaux 8, 9. L'angle y formé par les bords supérieurs 23 des branches 4, 5 peut être de l'ordre de 150 à 175°, plus spécifiquement de 160 à 170° (voir figure 10).

On décrit à présent les sangles 6, 7.

Les sangles 6, 7 sont réalisées en textile élastique et sont situées du côté extérieur de la ceinture 1. Elles sont agencées selon un V dont la pointe est dirigée vers le bas. L'angle δ formé par ce V peut par exemple être de l'ordre de 135 à 160°, plus spécifiquement de 145 à 150° (voir figure 10).

Chacune des sangles 6, 7 possède un bord médial 33 qui est sensiblement confondu avec l'axe médian transversal 2 et est fixé au bord médial 33 de l'autre sangle. De plus, chaque sangle 6, 7 possède un bord latéral 34 qui est fixé sur la branche 4, 5 correspondante, de préférence au voisinage de la partie extrême latérale 29 de celle-ci.

Les deux sangles 6, 7 sont fixées dans leur zone médiale sur la zone médiale de l'élément de liaison 15, en partie inférieure de celui-ci, par exemple au niveau des troisième et quatrième bandes 18, 19. De façon concrète, les bords médiaux 33 des sangles 6, 7 et 20 des bandes 18, 19 peuvent être assemblés entre eux. Les sangles 6, 7 passent du côté extérieur des éléments dorsaux 8, 9 sans être fixées à ces éléments 8, 9.

On décrit à présent la mise en place de la ceinture 1 sur une personne, en référence aux figures 4 à 9.

L'utilisateur place tout d'abord le dossard 3 contre son dos, les éléments dorsaux 8, 9 étant positionnés de part et d'autre du rachis lombaire, et les bords inférieurs 13 étant positionnés juste au-dessus du pli fessier. Les barres de rigidification 25 du premier ensemble sont alors situées de part et d'autre et à proximité immédiate des vertèbres.

De par leur disposition en V, avec un angle δ approprié, et leur positionnement sur la ceinture 1, les sangles 6, 7 passent sur les crêtes iliaques 36 de l'utilisateur, comme illustré sur la figure 4.

L'utilisateur dispose ainsi d'un certain nombre de repères anatomiques correspondant à différents constituants de la ceinture, ce qui conduit naturellement à un positionnement correct de la ceinture 1.

Plaçant ses doigts dans les lanières 35, l'utilisateur exerce alors une traction sur les parties extrêmes latérales 29 de la ceinture 1. L'inclinaison des lanières 35 par rapport à l'axe 2, c'est-à-dire en position d'utilisation sensiblement par rapport à l'axe du rachis, permettent de faciliter la traction car celle-ci s'effectue selon l'angle naturel pris par les avant-bras. De plus, cette inclinaison favorise la capacité de la ceinture 1 à se déformer comme cela est décrit maintenant.

Lorsque l'utilisateur pousse sur les lanières 35 - c'est-à-dire tire sur les parties extrêmes latérales 29 des branches 4, 5 de la ceinture 1 - dans l'axe des avant-bras pour mettre la ceinture en traction avant d'accrocher les deux parties extrêmes latérales 29 l'une sur l'autre dans sa zone abdominale, les mouvements suivants se produisent.

Le V formé par les éléments dorsaux 8, 9 s'ouvre, c'est-à-dire que l'angle α du V augmente pour atteindre α' qui peut par exemple être de l'ordre de 6 à 10°, avec α' > α + 1 ° (voir figure 11). En effet, du fait de la traction exercée, les bandes 16-19 se détendent, augmentant ainsi la distance entre les éléments dorsaux 8, 9 (orthogonalement à l'axe 2) ; de plus, l'agencement en V du montage favorise un écartement plus important dans la partie haute.

La traction exercée indirectement sur les sangles 6, 7 contribue à l'ouverture du V formé par les éléments dorsaux 8, 9 et provoque de plus un déplacement vers le haut du dossard 3, selon la flèche F1 illustrée sur les figures 6 et 11. Ce mouvement ascendant du dossard 3 est également rendu possible par le fait que les parties extrêmes latérales 29 des branches 4, 5 sont situées au-dessus du dossard 3. En outre, le fait que les sangles 6, 7 viennent en appui sur les crêtes iliaques 36 de l'utilisateur, occasionne un effet de coincement qui contribue à provoquer la montée du dossard 3. Les deux éléments dorsaux 8, 9 restent toutefois à la même hauteur l'un par rapport à l'autre.

La comparaison des figures 10 et 11 permet de mieux visualiser la façon dont la ceinture 1 se déforme et se déplace lorsqu'un utilisateur tire sur les parties extrêmes latérales 29.

On voit ainsi que l'angle α entre les deux éléments dorsaux 8, 9 augmente, et que l'angle y entre les branches 4, 5 augmente également, pour atteindre sensiblement 180°, les bords supérieurs 23 des branches 4, 5 étant alors sensiblement alignés et horizontaux. De plus, le dossard 3 se déplace vers le haut selon la flèche F1.

Sur les figures 10 et 11 est représentée la ligne imaginaire 37 reliant les bords supérieurs 32 des parties extrêmes latérales 29 des branches 4, 5. On voit que, à l'état non contraint, les bords supérieurs 12 des éléments dorsaux 8, 9 et les bords supérieurs 23 des branches 4, 5 sont situés sous cette ligne 37. En revanche, lorsque la ceinture 1 est mise en traction, les bords supérieurs 23 des branches 4, 5 sont situés sensiblement sur la ligne 37, tandis que les bords supérieurs 12 des éléments dorsaux 8, 9 sont situés au-dessus de la ligne 37.

Les conséquences de ces déplacements des éléments constitutifs de la ceinture 1 sont les suivants.

En position d'utilisation, comme illustré sur la figure 9, le dossard 3, qui s'est déplacé vers le haut, se situe au moins au droit des vertèbres lombaires L3, L4 et L5 et du segment intervertébral L5/S1 où se situe le disque vertébral le plus atteint par les lombalgies.

Ce mouvement ascendant du dossard 3 se traduit par un effort de poussée exercé du bas vers le haut sur l'utilisateur, dans sa zone lombaire. De plus, la traction exercée et l'ouverture du V formé par les deux éléments dorsaux 8, 9 provoque un placage du dossard 3 augmentant la pression des barres de rigidification 25, 26 dans les segments bas du rachis lombaire (L3/L4/L5) et sur le disque intervertébral L5/S1. Cette poussée, illustrée par la flèche F2 de la figure 9, s'exerce essentiellement en partie basse du dossard 3 du fait de l'agencement général en V et des sangles 6, 7 qui sont associées au dossard 3 en partie inférieure de celui-ci.

Ainsi, la traction circulaire et dans l'axe des avant-bras (c'est-à-dire globalement horizontale pour simplifier) exercée par l'utilisateur est transformée en une poussée dorsale inclinée du bas vers le haut et vers l'avant. Ceci contribue à atténuer la charge sur les segments bas du rachis lombaire et sur les segments intervertébraux bas.

Il va de soi que l'invention n'est pas limitée au mode de réalisation décrit ci-dessus à titre d'exemple mais qu'elle comprend tous les équivalents techniques et les variantes des moyens décrits ainsi que leurs combinaisons.

## Revendications

1. Ceinture de soutien lombaire présentant un axe de symétrie médian transversal (2), et comprenant :
- un dossard (3) destiné à être placé contre le dos d'un utilisateur et comportant deux éléments dorsaux (8, 9) distincts reliés par un élément de liaison (15) comprenant au moins une première et une deuxième bandes (16-19) de textile élastique, chaque bande (16-19) possédant un bord médial (20) qui, sensiblement confondu avec l'axe médian transversal (2), est fixé au bord médial (20) de l'autre bande, et un bord latéral (21) fixé au voisinage du bord médial (10) de l'élément dorsal (8, 9) correspondant ;
- deux branches (4, 5) de textile élastique destinées à être positionnées de part et d'autre de la partie inférieure du tronc de l'utilisateur, chacune desdites branches (4, 5) étant fixée par son bord médial (28) à un bord latéral (11) d'un élément dorsal (8, 9) et présentant une partie extrême latérale (29), les parties extrêmes latérales (29) des deux branches (4, 5) étant pourvues de moyens d'accrochage complémentaires pour pouvoir être assemblées lorsqu'elles sont placées l'une sur l'autre en regard de la zone abdominale de l'utilisateur, les deux branches (4, 5) et le dossard (3) formant un V aplati où les parties extrêmes latérales (29) des branches (4, 5) sont situées au-dessus du dossard (3), lorsque la ceinture (1) est à plat et à l'état non contraint ;
- deux sangles (6, 7) de textile élastique situées du côté extérieur de la ceinture (1) et agencées selon un V dont la pointe est dirigée vers le bas, chaque sangle (6, 7) possédant d'une part un bord médial (33) qui, sensiblement confondu avec l'axe médian transversal (2), est fixé au bord médial (33) de l'autre sangle, et d'autre part un bord latéral (34) fixé sur la branche (4, 5) correspondante, les deux sangles (6, 7) étant fixées dans leur zone médiale sur la zone médiale de l'élément de liaison (15), en partie inférieure de celui-ci ;
**caractérisée en ce que** les deux éléments dorsaux (8, 9) du dossard (3) sont agencés sensiblement selon un V dont la pointe est dirigée vers le bas, lorsque la ceinture (1) est à plat et à l'état non contraint, et **en ce que** les au moins une première et une deuxième bandes (16-19) de textile élastique forment les deux branches d'un V dont la pointe est dirigée vers le bas.

2. Ceinture selon la revendication 1, **caractérisée en ce que** le dossard (3) comprend au moins un premier ensemble de deux barres de rigidification (25) montées chacune sur un élément dorsal (8, 9), et agencées selon un V dont la pointe est dirigée vers le bas.

3. Ceinture selon la revendication 2, **caractérisée en ce que** le dossard (3) comprend un deuxième ensemble de deux barres de rigidification (26) montées chacune sur un élément dorsal (8, 9), et agencées selon un V dont la pointe est dirigée vers le bas, les barres (26) du deuxième ensemble étant disposées latéralement par rapport aux barres (25) du premier ensemble.

4. Ceinture selon la revendication 3, **caractérisée en ce que** l'angle (α1) formé par le V du premier ensemble de barres (25) est compris entre 6 et 8°, et **en ce que** l'angle (α2) formé par le V du deuxième ensemble de barres (26) est compris entre 8 et 10°, lorsque la ceinture (1) est à plat et à l'état non contraint.

5. Ceinture selon l'une des revendications 1 à 4, **caractérisée en ce que** les première et deuxième bandes (16, 17) de l'élément de liaison (15) sont situées en portion supérieure de l'élément de liaison (15) et **en ce que** l'élément de liaison (15) comprend, en portion inférieure, une troisième et une quatrième bandes (18, 19) de textile élastique formant les deux branches d'un V dont la pointe est dirigée vers le bas, chacune des troisième et quatrième bandes (18, 19) possédant un bord médial (20) qui, sensiblement confondu avec l'axe médian transversal (2), est fixé au bord médial (20) de l'autre bande, et un bord latéral (21) fixé au bord médial de l'élément dorsal (8, 9) correspondant.

6. Ceinture selon l'une des revendications 1 à 5, **caractérisée en ce que** chaque branche (4, 5) est formée par l'assemblage de façon décalée d'au moins deux bandes (30, 31) sensiblement rectangulaires, de sorte que la largeur de la branche (4, 5) diminue depuis l'élément dorsal (3) en direction de la partie extrême latérale (29).

7. Ceinture selon l'une des revendications 1 à 6, **caractérisée en ce que** les sangles (6, 7) sont positionnées sur la ceinture (1) de sorte à venir en appui sur les crêtes iliaques (36) de l'utilisateur, en position d'utilisation de la ceinture (1).

8. Ceinture selon l'une des revendications 1 à 5, **caractérisée en ce que** les parties extrêmes latérales (29) des branches (4, 5) comprennent des moyens de réception (35) des doigts de l'utilisateur qui, lorsque la ceinture (1) est à plat et à l'état non contraint, sont disposés de façon inclinée par rapport à l'axe médian transversal (2) d'un angle (β) compris entre 20 et 40°, par exemple voisin de 30°, du bas vers le haut lorsque l'on s'éloigne dudit axe (2).

## Patentansprüche

1. Lendenstützgurt, der eine mittlere Quersymmetrieachse (2) aufweist und Folgendes umfasst:
- einen Latz (3), der dazu vorgesehen ist, gegen den Rücken eines Benutzers platziert zu werden, und zwei verschiedene dorsale Elemente (8, 9) umfasst, die durch ein Bindeelement (15) verbunden sind, das mindestens einen ersten und einen zweiten Streifen (16-19) aus elastischem Gewebe umfasst, wobei jeder Streifen (16-19) über eine mittlere Kante (20), die im Wesentlichen mit der mittleren Quersymmetrieachse (2) zusammenfallend an der mittleren Kante (20) des anderen Streifens befestigt ist, und eine seitliche Kante (21), die in der Nähe der mittleren Kante (10) des entsprechenden dorsalen Elements (8, 9) befestigt ist, verfügt;
- zwei Arme (4, 5) aus elastischem Gewebe, die dazu vorgesehen sind, zu beiden Seiten des unteren Teils des Rumpfs des Benutzers positioniert zu werden, wobei jeder der besagten Arme (4, 5) durch seine mittlere Kante (28) an einer seitlichen Kante (11) eines dorsalen Elements (8, 9) befestigt ist und einen äußersten seitlichen Teil (29) aufweist, wobei die äußersten seitlichen Teile (29) der zwei Arme (4, 5) mit komplementären Kopplungsmitteln versehen ist, um sie zusammenzufügen, wenn sie aufeinander in Bezug auf den Abdominalbereich des Benutzers platziert werden, wobei die zwei Arme (4, 5) und der Latz (3) ein flaches V bilden, in dem die äußersten seitlichen Teile (29) der Arme (4, 5) sich über dem Latz (3) befinden, wenn der Gurt (1) flach und im ungespannten Zustand ist;
- zwei Spanngurte (6, 7) aus elastischem Gewebe, die sich auf der Außenseite des Gurts (1) befinden und gemäß einem V eingerichtet sind, dessen Spitze nach unten gerichtet ist, wobei jeder Spanngurt (6, 7) über einerseits eine mittlere Kante (33), die im Wesentlichen mit der mittleren Quersymmetrieachse (2) zusammenfallend an der mittleren Kante (33) des anderen Spanngurts befestigt ist, und andererseits eine seitliche Kante (34), die auf dem jeweiligen Arm (4, 5) befestigt ist, verfügt, wobei die zwei Spanngurte (6, 7) in ihrem mittleren Bereich auf dem mittleren Bereich des Bindeelements (15) im unteren Teil dieses befestigt ist;
**dadurch gekennzeichnet, dass** die zwei dorsalen Elemente (8, 9) des Latzes (3) im Wesentlichen gemäß einem V eingerichtet sind, dessen Spitze nach unten gerichtet ist, wenn der Gurt (1) flach und im ungespannten Zustand ist, und dass der mindestens eine erste und eine zweite Streifen (16-19) aus elastischem Gewebe die zwei Arme eines V bilden, dessen Spitze nach unten gerichtet ist.

2. Gurt nach Anspruch 1, **dadurch gekennzeichnet, dass** der Latz (3) mindestens eine erste Einheit von zwei Versteifungsstäben (25) umfasst, die jeweils auf einem dorsalen Element (8, 9) montiert sind und gemäß einem V eingerichtet sind, dessen Spitze nach unten gerichtet ist.

3. Gurt nach Anspruch 2, **dadurch gekennzeichnet, dass** der Latz (3) eine zweite Einheit von zwei Versteifungsstäben (26) umfasst, die jeweils auf einem dorsalen Element (8, 9) montiert sind und gemäß einem V eingerichtet sind, dessen Spitze nach unten gerichtet ist, wobei die Stäbe (26) der zweiten Einheit seitlich in Bezug auf die Stäbe (25) der ersten Einheit angeordnet sind.

4. Gurt nach Anspruch 3, **dadurch gekennzeichnet, dass** der Winkel (α1), der von dem V der ersten Einheit von Stäben (25) gebildet wird, zwischen 6 und 8° liegt und dass der Winkel (α2), der von dem V der zweiten Einheit von Stäben (26) gebildet wird, zwischen 8 und 10° liegt, wenn der Gurt (1) flach und im ungespannten Zustand ist.

5. Gurt nach einem der Ansprüche 1 bis 4, **dadurch gekennzeichnet, dass** der erste und der zweite Streifen (16, 17) des Bindeelements (15) sich in einem oberen Teil des Bindeelements (15) befinden und dass das Bindeelement (15) in einem unteren Teil einen dritten und einen vierten Streifen (18, 19) aus elastischem Gewebe umfasst, die die zwei Arme eines V bilden, dessen Spitze nach unten gerichtet ist, wobei jeder des dritten und des vierten Streifens (18, 19) über eine mittlere Kante (20), die im Wesentlichen mit der mittleren Quersymmetrieachse (2) zusammenfallend an der mittleren Kante (20) des anderen Streifens befestigt ist, und eine seitliche Kante (21), die an der mittleren Kante des entsprechenden dorsalen Elements (8, 9) befestigt ist, verfügt.

6. Gurt nach einem der Ansprüche 1 bis 5, **dadurch gekennzeichnet, dass** jeder Arm (4, 5) durch das versetzte Zusammenfügen von mindestens zwei im Wesentlichen rechteckigen Streifen (30, 31) gebildet wird, so dass die Breite des Arms (4, 5) von dem dorsalen Element (3) in Richtung des äußersten seitlichen Teils (29) abnimmt.

7. Gurt nach einem der Ansprüche 1 bis 6, **dadurch gekennzeichnet, dass** die Spanngurte (6, 7) auf dem Gurt (1) so positioniert werden, dass sie auf den Darmbeinkämmen (36) des Nutzers in einer Position der Verwendung des Gurts (1) aufliegen.

8. Gurt nach einem der Ansprüche 1 bis 5, **dadurch gekennzeichnet, dass** die äußersten seitlichen Teile (29) der Arme (4, 5) Aufnahmemittel (35) für die Finger des Benutzers umfassen, die, wenn der Gurt (1) flach und im ungespannten Zustand ist, geneigt in Bezug auf die mittlere Quersymmetrieachse (2) in einem Winkel (β) zwischen 20 und 40°, beispielsweise nahe 30°, von unten nach oben angeordnet sind, wenn man sich von der besagten Achse (2) entfernt.

## Claims

1. A lumbar support belt having a transverse median axis of symmetry (2), and comprising:
- a back pad (3) intended to be placed against the back of a user and including two separate dorsal members (8, 9) linked by a linking member (15) comprising at least one first and one second elastic textile strips (16-19), each strip (16-19) having a medial edge (20) which substantially coincides with the transverse median axis (2) and which is fastened to the medial edge (20) of the other strip, and a lateral edge (21) fastened in the vicinity of the medial edge (10) of the corresponding dorsal member (8, 9);
- two elastic textile arms (4, 5) intended to be positioned on either side of the lower part of the trunk of the user, each of said arms (4, 5) being fastened by its medial edge (28) to a lateral edge (11) of a dorsal member (8, 9) and having a lateral extreme portion (29), the lateral extreme portions (29) of the two arms (4, 5) being provided with complementary attachment means to be able to be assembled when they are placed one over the other facing the abdominal area of the user, the two arms (4, 5) and the back pad (3) forming a flattened V where the lateral extreme portions (29) of the arms (4, 5) are located above the back pad (3), when the belt (1) is laid flat and in the unstressed condition;
- two elastic textile straps (6, 7) located at the outer side of the belt (1) and arranged in a V whose tip is downwardly directed, each strap (6, 7) having on the one hand a medial edge (33) which substantially coincides with the transverse median axis (2) and which is fastened to the medial edge (33) of the other strap, and on the other hand a lateral edge (34) fastened to the corresponding arm (4, 5), the two straps (6, 7) being fastened in their medial area on the medial area of the linking member (15), in the lower part thereof;
**characterized in that** the two dorsal members (8, 9) of the back pad (3) are substantially arranged in a V whose tip is downwardly directed, when the belt (1) is laid flat and in the unstressed condition, and **in that** the at least first and second elastic textile strips (16-19) form the two arms of a V whose tip is downwardly directed.

2. The belt according to claim 1, **characterized in that** the back pad (3) comprises at least one first assembly of two stiffening bars (25) each mounted on a dorsal member (8, 9), and arranged in a V, whose tip is downwardly directed.

3. The belt according to claim 2, **characterized in that** the back pad (3) comprises a second assembly of two stiffening bars (26) each mounted on a dorsal member (8, 9), and arranged in a V whose tip is downwardly directed, the bars (26) of the second assembly being laterally disposed relative to the bars (25) of the first assembly.

4. The belt according to claim 3, **characterized in that** the angle (α1) formed by the V of the first assembly of bars (25) is comprised between 6 and 8° and **in that** the angle (α2) formed by the V of the second assembly of bars (26) is comprised between 8 and 10°, when the belt (1) is laid flat and in the unstressed condition.

5. The belt according to any of claims 1 to 4, **characterized in that** the first and second strips (16, 17) of the linking member (15) are located in upper portion of the linking member (15) and **in that** the linking member (15) comprises, in lower portion, a third and a fourth elastic textile strips (18, 19) forming the two arms of a V whose tip is downwardly directed, each of the third and fourth strips (18, 19) having a medial edge (20) which substantially coincides with the transverse median axis (2) and which is fastened to the medial edge (20) of the other strip, and a lateral edge (21) fastened to the medial edge of the corresponding dorsal member (8, 9).

6. The belt according to any of claims 1 to 5, **characterized in that** each arm (4, 5) is formed by the assembly in a shifted manner of at least two substantially rectangular strips (30, 31), so that the width of the arm (4, 5) decreases from the dorsal member (3) towards the lateral extreme portion (29).

7. The belt according to any of claims 1 to 6, **characterized in that** the straps (6, 7) are positioned on the belt (1) so as to bear on the iliac crests (36) of the user, in the position of use of the belt (1).

8. The belt according to any of claims 1 to 5, **characterized in that** the lateral extreme portions (29) of the arms (4, 5) comprise receiving means (35) of the fingers of the user which, when the belt (1) is laid flat and in the unstressed condition, are disposed in a tilted manner relative to the transverse median axis (2) by an angle (β) comprised between 20 and 40°, for example close to 30°, from the bottom to the top when going away from said axis (2).
